# EUROPÄISCHE PATENTSCHRIFT

(11) **EP 2 217 587 B1**
(45) Veröffentlichungstag und Bekanntmachung des Hinweises auf die Patenterteilung: **23.09.2015**
(21) Anmeldenummer: 08847460.6
(22) Anmeldetag: 28.10.2008
(51) Int. Cl.: C07D 313/04

(54) **VERFAHREN ZUR HERSTELLUNG VON E-CAPROLACTON**
METHOD FOR PRODUCING E-CAPROLACTONE
PROCÉDÉ DE PRODUCTION D' E-CAPROLACTONE

(30) Priorität: 05.11.2007 US 985424 P
(43) Veröffentlichungstag der Anmeldung: 18.08.2010
(73) Patentinhaber: BASF SE, 67056 Ludwigshafen (DE)
(72) Erfinder: PINKOS, Rolf, 67098 Bad Dürkheim (DE); TEBBEN, Gerd-Dieter, 68239 Mannheim (DE); KRUG, Thomas, 67550 Worms (DE); SIRCH, Tilman, 67105 Schifferstadt (DE); SPENGEMAN, Todd C, Sugar Land, TX 77459 (US); BEJUNE, Stephanie, Angleton TX 77515 (US); ANDRESS, Jeffrey T., Lake Jackson TX 77566 (US); GASIOROWSKI, Todd, Lake Jackson TX 77566 (US)
(86) Internationale Anmeldenummer: PCT/EP2008/064608
(87) Internationale Veröffentlichungsnummer: WO 2009/059913

(56) Entgegenhaltungen:
- WO-A-97/31883
- DE-A1- 10 100 552
- DE-A1- 10 308 489
- GB-A- 1 140 184

## Beschreibung

Die Erfindung betrifft ein verbessertes Verfahren zur Herstellung von ε-Caprolacton in einer Reinheit über 99% durch Cyclisierung von 6-Hydroxycapronsäureester in der Flüssigphase bei 150 bis 400°C und 1 bis 1020 hPa abs., Abtrennung und Kondensation der unter Cyclisierungsbedingungen flüchtigen Verbindungen, bei dem das zurückbleibende Sumpfprodukt der Cyclisierung in mindestens einem weiteren Reaktor thermisch in Gegenwart von 0,1 bis 90 Gew.-% Mono-, Di- oder Polyolen behandelt wird, flüchtige Verbindungen abgetrennt und kondensiert werden und ε-Caprolacton durch Destillation aus den Kondensaten gewonnen wird.

ε-Caprolacton bzw. die daraus durch Polyaddition hergestellten Polycaprolactone dienen zur Herstellung von Polyurethanen.

Es ist bekannt, 6-Hydroxycapronsäureester in der Gas- oder Flüssigphase zu Caprolacton zu cyclisieren. So beschreibt DE 38 23 213 die Cyclisierung von 6-Hydroxycapronsäureestern zu Caprolacton in der Gasphase in Gegenwart oxidischer Katalysatoren und inerter Trägergase.

Weiterhin ist aus WO 97/31883 ein Verfahren zur Herstellung von 1,6-Hexandiol und ε-Caprolacton aus einem Adipinsäure, 6-Hydroxycapronsäure und geringe Mengen 1,4-Cyclohexandiole enthaltenden Carbonsäuregemisch bekannt, das als Nebenprodukte der Oxidation von Cyclohexan zu Cyclohexanon/ Cyclohexanol mit Sauerstoff oder Sauerstoff enthaltenden Gasen und durch Wasserextraktion des Reaktionsgemisches erhalten wird.

Die wässrigen Lösungen von Carbonsäuren, die bei der Oxidation von Cyclohexan zu Cyclohexanol und Cyclohexanon (vgl. Ullmann's Encyclopedia of Industrial Chemistry, 5. Ed., 1987, Vol. A8, S. 49) als Nebenprodukte entstehen im Folgenden Dicarbonsäurelösung (DCL) genannt, enthalten (berechnet wasserfrei in Gew.-%) im Allgemeinen zwischen 10 und 40 % Adipinsäure, zwischen 10 und 40 % 6-Hydroxycapronsäure, zwischen 1 und 10 % Glutarsäure, zwischen 1 und 10 % 5-Hydroxyvaleriansäure, zwischen 1 und 5 % 1,2-Cyclohexandiole, zwischen 1 und 5 % 1,4-Cyclohexandiole, zwischen 2 und 10 % Ameisensäure sowie eine Vielzahl weiterer Mono- und Dicarbonsäuren, Ester, Oxo- und Oxa-Verbindungen, deren Einzelgehalte im Allgemeinen 5 % nicht übersteigen. Beispielsweise seien Essigsäure, Propionsäure, Buttersäure, Valeriansäure, Capronsäure, Oxalsäure, Malonsäure, Bernsteinsäure, 4-Hydroxybuttersäure und gamma-Butyrolacton genannt.

Die wässrigen Lösungen werden nach Entwässerung mit einem niedermolekularen Alkohol zu den entsprechenden Carbonsäureestern verestert, das erhaltene Veresterungsgemisch wird in einer ersten Destillationsstufe von überschüssigem Alkohol und

Leichtsiedern befreit. Aus dem Sumpfprodukt wird in einer zweiten Destillationsstufe eine Auftrennung in eine von 1,4-Cyclohexandiolen im Wesentlichen freie Esterfraktion und zumindest den größeren Teil der Cyclohexandiole enthaltende Fraktion erhalten. Durch eine dritte Destillationsstufe wird eine im Wesentlichen 6-Hydroxycapronsäureester enthaltende Fraktion (Stufe 2) gewonnen und in der Gas-, bevorzugt in der Flüssigphase, zu ε-Caprolacton cyclisiert.

Der im Wesentlichen 6-Hydroxycapronsäureester enthaltende Strom wird bei vermindertem Druck auf Temperaturen über 200 °C erhitzt, wodurch 6-Hydroxycapronsäureester zu Caprolacton cyclisiert werden und aus dem Cyclisierungsprodukt durch Destillation reines Caprolacton gewonnen wird. Die Flüssigphasen-Cyclisierung kann ohne Katalysator erfolgen, wird aber bevorzugt in Anwesenheit eines Katalysators durchgeführt. In manchen Fällen ist es vorteilhaft, die Cyclisierungs-Reaktion in Gegenwart von hochsiedenden Mono-, Di- oder Polyolen durchzuführen.

Die Reaktionsprodukte, vorwiegend Caprolacton und Veresterungsalkohol, werden gasförmig aus dem Reaktionsgemisch entfernt und kondensiert. Durch fraktionierende Destillation wird aus dem Kondensat Caprolacton gewonnen.

Nachteilig ist, dass insbesondere wenn das Verfahren im industriellen Maßstab durchgeführt wird, zur Vermeidung von sehr langen Verweilzeiten und dadurch bedingtem teurem Reaktionsraum lediglich Caprolacton-Ausbeuten von bis zu 90 %, in der Regel nur bis zu 80 % erzielt werden. Dies ist unter anderem auf Oligomerisierungs- und Polymerisations-Nebenreaktionen zurückzuführen, bei denen 6-Hydroxycapronsäureester der Formel HO-(CH₂)₅-COOR (R ist z.B. ein C₁- bis C₄-Alkylrest) Dimere der Formel HOCH₂-(CH₂)₄-COO-(CH₂)₅-COOR bilden, aus denen durch Umsetzung mit weiteren Hydroxyestern Oligomere und Polymere entstehen können.

Enthält der 6-Hydroxycapronsäureester noch Diester, insbesondere Adipinsäurediester, die auch ungesättigt sein können, so kann sich z.B. im Falle der Dimethylester der dimere Ester CH₃OOC-(CH₂)₄-COO(CH₂)₅-COOCH₃ bilden, aus dem ebenfalls unter Einbau weiterer 6-Hydroxyester-Moleküle Oligomere und Polymere entstehen können. In Anwesenheit dieser Diester, insbesondere wenn sie Gehalte von 1 % übersteigen, reduziert sich die Ausbeute an Caprolacton, insbesondere bei kurzen Verweilzeiten, erheblich, teilweise unter 70 %.

Es bestand daher die Aufgabe, ein Verfahren zur Flüssigphasen-Herstellung von ε-Caprolacton in einer Reinheit von mehr als 99 % ausgehend von 6-Hydroxycapronsäureestern oder diese enthaltenden Gemischen bereitzustellen, bei dem, insbesondere bei der industriellen Durchführung, höhere Caprolacton-Ausbeuten (bezogen auf die bei der Cyclisierung eingesetzten 6-Hydroxycapronsäureester) erzielt werden.

Diese Aufgabe wurde gelöst mit einem Verfahren zur Herstellung von ε-Caprolacton in einer Reinheit über 99 % durch Cyclisierung von 6-Hydroxycapronsäure-ester in der Flüssigphase bei 150 bis 400°C und 1 bis 1020 hPa abs., Abtrennung und Kondensation der unter Cyclisierungsbedingungen flüchtigen Verbindungen, dadurch gekennzeichnet, dass das zurückbleibende Sumpfprodukt der Cyclisierung in mindestens einem weiteren Reaktor thermisch in Gegenwart von 0,1 bis 90 Gew.-% Mono-, Di- oder Polyolen behandelt wird, flüchtige Verbindungen abgetrennt und kondensiert werden und ε-Caprolacton durch Destillation aus den Kondensaten gewonnen wird.

In dem weiteren Reaktor oder den Reaktoren kann die thermische Behandlung unter den Druck-und Temperaturbedingungen der Cyclisierung erfolgen, bevorzugt wird aber bei höherer Temperatur, beispielsweise bei 50°C höherer Temperatur, thermisch behandelt. Es kann auch zusätzlich der Druck variiert werden, bevorzugt wird ein geringerer Druck als in der Cyclisierung eingestellt.

Die Sumpfprodukte der Caprolacton-Herstellung stellen komplexe Gemische aus oligomeren und polymeren Estern dar, die im Allgemeinen aus 6-Hydroxycapronsäure-, Adipinsäure-, ungesättigten Adipinsäure- und Dioleinheiten wie 1,4-Cyclohexandiole enthalten. Es können aber auch noch andere Alkoholkomponenten wie z.B. 1,5-Pentandiol, 1,6-Hexandiol, 1,4-Cyclohexandimethanol, Nonanol, Tridecanol oder Pentadecanol enthalten, die entweder vorher zugesetzt wurden und/oder systemimmanent sein können, wie z.B. 1,5-Pentandiol.

Es war daher überraschend, dass sich aus den Sumpfprodukten der Cyclisierung zu ε-Caprolacton durch Erhitzen noch zusätzliche, bemerkenswert große Mengen an ε-Caprolacton gewinnen lassen und dass zusätzlich die hohen Reinheitsanforderungen an das Produkt von Reinheiten > 99 % erfüllt werden können.

Erfindungsgemäß wird das unter den Bedingungen der Cyclisierung nicht flüchtige Sumpfprodukt der 6-Hydroxycapronsäureester-Cyclisierung in mindestens einem weiteren, d.h. vom Cyclisierungsreaktor verschiedenen, Reaktor diskontinuierlich oder kontinuierlich einer thermischen Behandlung unterworfen. Die dabei flüchtigen, abdestillierenden Verbindungen werden kondensiert. Aus dem Kondensat wird durch Destillation zusätzliches Caprolacton gewonnen, das die Caprolacton-Gesamtausbeute steigert.

Die thermische Behandlung erfolgt bei Temperaturen von 150 bis 400°C, bevorzugt 180 bis 350°C, besonders bevorzugt 190 bis 330°C, und Drücken von 1 bis 1020 hPa abs., bevorzugt 2 bis 500 hPa, besonders bevorzugt 5 bis 200 hPa. Die thermische Behandlung des Sumpfproduktes der Cyclisierung erfolgt im Allgemeinen bei der gleichen oder bevorzugt bei einer bis zu 100°C höheren Temperatur wie die Cyclisierung der 6-Hydroxycapronsäureester durchgeführt werden. Es ist besonders bevorzugt, die thermische Behandlung des Sumpfprodukts aus der Cyclisierung bei bis zu 50°C, und insbesondere bevorzugt bei bis zu 30°C höheren Temperaturen als bei der 6-Hydroxycapronsäureester-Cyclisierung durchzuführen. Die thermische Behandlung kann kontinuierlich oder diskontinuierlich erfolgen. Dabei beträgt die Verweilzeit im Allgemeinen von 0,1 bis zu 24 Stunden, bevorzugt sind bis zu 15 Stunden, besonders bevorzugt bis zu 10 Stunden. Der Reaktionsdruck kann dem Druck der Cyclisierung entsprechen, ein niedrigerer Druck ist jedoch bevorzugt. Absolut liegt er bevorzugt unter 50 hPa, besonders bevorzugt zwischen 1 und 30 hPa.

Als Reaktoren können für die thermische Behandlung beispielsweise durchmische Behälter mit aufgesetzter Destillationskolonne, Wischblattverdampfer (Sambayverdampfer) oder Fallfilmverdampfer verwendet werden. Es ist weiterhin bevorzugt, die gasförmigen Reaktionsprodukte der thermischen Behandlung nicht einstufig auszutreiben, sondern eine Trennvorrichtung mit mindestens einem theoretischen Boden und teilweisem flüssigen Produktrücklauf zu verwenden. Dies steigert überraschend die Ausbeute an Caprolacton. Es ist auch möglich, die thermische Behandlung in einer Reaktionskolonne durchzuführen.

Dem Sumpfprodukt der Cyclisierung kann ein Lösungsmittel zugesetzt werden.

In einer bevorzugten Ausführungsform wird dem Sumpfprodukt der Cyclisierung vor oder während der thermischen Behandlung hochsiedendes Mono-, Di- oder Polyol zugesetzt. Unter hochsiedend werden dabei Mono-, Di- oder Polyole mit Siedepunkten bevorzugt oberhalb des Siedepunkts von Caprolacton unter den gegebenen Reaktionsdrücken verstanden. Als Mono-, Di- oder Polyol kann Decanol, Undecanol, Tridecanol, Pentadecanol, Ocatadecanol, 1,4-Butandiol, 1,5-Pentandiol, 1,6-Hexandiol, 1,4-Cyclohexandiole, Butylethylpropandiol, Neopentylglykol, Triethylenglykol, Tetraethylenglykol, Trimethylolpropan oder Glycerin verwendet werden. Geeignet sind ebenfalls hochsiedende Mono-, Di- oder Polyol-enthaltende Gemische, die als Destillationsrückstände bei der Synthese von Mono-, Di- oder Polyol anfallen, z.B. Destillationsrückstände die bei der Synthese von Nonanol oder Tridekanol/Pentadecanol erhalten werden. Diese hochsiedenden Mono-, Di- oder Polyole werden vorgelegt und/oder dem Reaktionsgemisch zugesetzt oder separat zudosiert z.B. jeweils in Konzentrationen von 0,1 bis 90 Gew.-%, bevorzugt 1 bis 60 Gew.-%, besonders bevorzugt 5 bis 30 Gew-%.

In einer weiteren bevorzugten Ausführungsform wird die thermische Behandlung des Sumpfprodukts der Cyclisierung in Gegenwart eines Katalysators durchgeführt. Dabei kann, v.a. bei Verwendung eines homogen gelösten Katalysator in der Cyclisierung, dieser weiterhin in der thermischen Behandlung vorhanden sein, es kann aber auch ein weiterer hinzugefügt werden.

Als Katalysatoren sind für die thermische Behandlung die an sich auch für die Cyclisierung der 6-Hydroxycapronsäureester bekannten Katalysatoren geeignet. Als Katalysatoren eignen sich Katalysatoren, die homogen gelöst oder heterogen vorliegen können. Beispiele sind Alkali- und Erdalkalimetallhydroxide-, -oxide,-carbonate, -alkoxylate oder -carboxylate, Lewissäuren oder Lewisbasen, bevorzugt aus der III. und IV. Hauptgruppe bzw. der I. bis VIII. Nebengruppe des Periodensystems der Elemente oder Oxide von seltenen Erdmetallen oder deren Gemische. Beispielhaft seien genannt Magnesiumoxid, Zinkoxid, Bortrioxid, Titandioxid, Siliciumdioxid, Zinndioxid, Wismutoxid, Kupferoxid, Lanthanoxid, Zirkondioxid, Vanadiumoxide, Chromoxide, Wolframoxide, Eisenoxide, Ceroxid, Aluminiumoxid, Hafniumoxid, Bleioxid, Antimonoxid, Bariumoxid, Calciumoxid, Natriumhydroxid, Kaliumhydroxid, Neodymoxid. Es können auch Mischungen von Oxiden eingesetzt werden, wobei es sich um Mischungen der Einzelkomponenten handeln kann oder auch um Mischoxide wie sie z.B. in Zeolithen, Tonerden oder Heteropolysäuren vorkommen. Bevorzugt sind Lewissäuren oder -basen auf Basis von Aluminium, Zirkon oder Titan.

Vorteilhaft hält man eine Katalysatorbelastung von 0,01 bis 40, bevorzugt 0,05 bis 20, insbesondere 0,07 bis 10 g Edukt (6-Hydroxycapronsäureester) je g Katalysator und Stunde ein. Homogene Katalysatoren werden in einer Konzentration von üblicherweise 10 bis 10.000 ppm, bevorzugt 5 bis 5000 ppm, besonders bevorzugt 100 bis 1000 ppm, verwendet.

Während der thermischen Behandlung werden flüchtige Verbindungen als Kopfprodukte in Form von Gemischen erhalten, die als Hauptprodukt ε-Caprolacton neben Leichtsieder wie z.B. niederen Alkohole enthalten.

Es kann zur weiteren Steigerung der ε-Caprolacton-Ausbeute sinnvoll sein, das Sumpfprodukt der thermischen Behandlung zurückzuführen oder einer weiteren getrennten thermischen Behandlung zu unterwerfen.

Im erfindungsgemäßen Verfahren wird für die thermische Behandlung das Sumpfprodukt der Cyclisierung der 6-Hydroxycapronsäureester zu ε-Caprolacton verwendet.

Als 6-Hydroxycapronsäureester kommen in der Regel Ester der Alkanole mit 1 bis 12 C-Atomen, Cycloalkanole mit 5 bis 7 C-Atomen, Aralkanole mit 7 bis 8 C-Atomen oder Phenole mit 6 bis 8 C-Atomen in Betracht. Dabei können Methanol, Ethanol, Propanol, iso-Propanol, n- oder i-Butanol oder auch n-Pentanol oder i-Pentanol oder Gemische der Alkohole, bevorzugt aber Alkohole mit 1 bis 4 C-Atomen, besonders bevorzugt Methanol verwendet werden. Auch Diole wie Butandiol oder Pentandiol kommen prinzipiell in Betracht. Die Estergruppen in den 6-Hydroxycapronsäureestern können gleich oder verschieden sein. Das besonders bevorzugte Edukt ist 6-Hydroxycapronsäuremethylester.

Die Herstellung der 6-Hydroxycapronsäureester kann beispielsweise gemäß DE-A 197 50 532, auf die hier ausdrücklich Bezug genommen wird und hier als inkorporiert gelten soll, erfolgen.

Gemäß DE-A 197 50 532 wird 6-Hydroxycapronsäureester durch katalytische Hydrierung von Adipinsäurediestern oder Eduktströmen, die diese Ester als wesentliche Bestandteile enthalten, Destillation des Hydrieraustrags und Abtrennung von Hexandiol und Adipinsäurediestern gewonnen.

Dabei wird die Hydrierung bevorzugt in der Flüssigphase durchgeführt. Als Hydrierkatalysatoren finden in diesem Verfahren im Allgemeinen heterogene, aber auch homogene, zur Hydrierung von Carbonylgruppen geeignete Katalysatoren Verwendung. Sie können sowohl fest angeordnet als auch mobil, z.B. in einem Wirbelbettreaktor, eingesetzt werden. Beispiele hierfür sind z.B. in Houben-Weyl, "Methoden der Organischen Chemie", Band IV/1c, S. 16 bis 26 beschrieben. Von den zu verwendenden Hydrierkatalysatoren sind solche bevorzugt, die ein oder mehrere Elemente der Gruppe I b, VI b, VII b und VIII b sowie III a, IV a und V a des Periodensystems der Elemente, insbesondere Kupfer, Chrom, Rhenium, Kobalt, Rhodium, Nickel, Palladium, Eisen, Platin, Indium, Zinn und/oder Antimon enthalten. Besonders bevorzugt sind Katalysatoren, die Kupfer, Kobalt und/oder Rhenium enthalten.

Weiterhin kann die Herstellung der 6-Hydroxycapronsäureester gemäß WO 97/31883, auf die hier ausdrücklich Bezug genommen wird und hier als inkorporiert gelten soll, erfolgen.

Die Herstellung der 6-Hydroxycapronsäureester erfolgt nach WO 97/31883 derart, dass ein Adipinsäure, 6-Hydroxycapronsäure und geringe Mengen 1,4-Cyclohexandiole enthaltendes Carbonsäuregemisch, das als Nebenprodukt der Oxidation von Cyclohexan zu Cyclohexanon/Cyclohexanol mit Sauerstoff oder Sauerstoff enthaltenden Gasen durch Wasserextraktion des Reaktionsgemisches erhältlich ist, mit einem niedermolekularen Alkohol zu den entsprechenden Carbonsäureestern verestert wird, und das so erhaltene Veresterungsgemisch in mindestens einer Destillationsstufe aufgetrennt wird.

In einer bevorzugten Ausführungsform wird 6-Hydroxycapronsäuremethylester erhalten, in dem
- das erhaltene Veresterungsgemisch in einer ersten Destillationsstufe von überschüssigem Methanol und Leichtsiedern befreit wird,
- aus dem Sumpfprodukt in einer zweiten Destillationsstufe eine Auftrennung in eine von 1,4-Cyclohexandiolen im Wesentlichen freie Esterfraktion und eine zumindest den größeren Teil der 1,4-Cyclohexandiole enthaltende Fraktion durchgeführt wird und ein
- 6-Hydroxycapronsäuremethylester-Strom von der Esterfraktion in einer dritten Destillationsstufe abgetrennt wird.

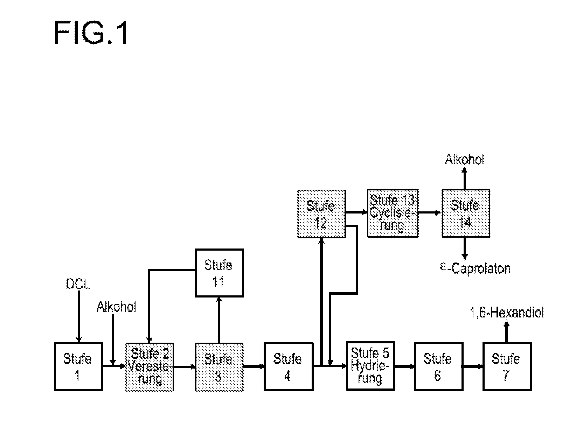

Zum besseren Verständnis wird das Verfahren zur Herstellung von ε-Caprolacton entsprechend WO 97/31883 gemäß Figur 1 erläutert, in der die einzelnen Verfahrensschritte in weitere Stufen aufgeschlüsselt sind, wobei die Stufen 2, 3, 4 sowie 12, 13 und 14 für das Verfahren zur Herstellung von ε-Caprolacton essentiell sind und die Stufen 3 und 4 auch zusammengefasst werden können.

Die Dicarbonsäurelösung (DCL) ist im Allgemeinen eine wässrige Lösung mit einem Wasseranteil von 20 bis 80 %. Da eine Veresterungsfraktion eine Gleichgewichtsreaktion darstellt, bei der Wasser entsteht, ist es sinnvoll, insbesondere bei Veresterung mit z.B. Methanol vorhandenes Wasser vor der Reaktion zu entfernen, vor allem wenn während der Veresterungsreaktion Wasser nicht, z.B. nicht azeotrop, entfernt werden kann. Die Entwässerung in Stufe 1 kann z.B. mit einem Membransystem erfolgen, oder bevorzugt durch eine Destillationsapparatur, bei der bei 10 bis 250°C, bevorzugt 20 bis 200°C, besonders bevorzugt 30 bis 200°C und einem Druck von 1 bis 1500 hPa, bevorzugt 5 bis 1100 hPa, besonders bevorzugt 20 bis 1000 hPa Wasser über Kopf und höhere Monocarbonsäuren, Dicarbonsäuren und 1,4-Cyclohexandiole über Sumpf abgetrennt werden. Die Sumpftemperatur wird dabei bevorzugt so gewählt, dass das Sumpfprodukt flüssig abgezogen werden kann. Der Wassergehalt im Sumpf der Kolonne kann 0,01 bis 10 Gew.-%, bevorzugt 0,01 bis 5 Gew.-%, besonders bevorzugt 0,01 bis 1 Gew.-% betragen.

Die Abtrennung des Wassers kann so erfolgen, dass das Wasser überwiegend säurefrei erhalten wird oder man kann die in der DCL enthaltenen niederen Monocarbonsäuren - im Wesentlichen Ameisensäure -- zum größten Teil mit dem Wasser abdestillieren, damit diese in der Veresterung keinen Veresterungsalkohol binden.

Dem Carbonsäurestrom aus der Stufe 1 wird Alkohol ROH mit 1 bis 10 C-Atomen zugemischt. Dabei können Methanol, Ethanol, Propanol oder i-Propanol oder Gemische der Alkohole, bevorzugt aber Methanol einerseits oder C₄ und höhere Alkohole, insbesondere mit 4 bis 8 C-Atomen und bevorzugt n- oder i-Butanol oder auch n-Pentanol oder i-Pentanol andererseits verwendet werden. Das Mischungsverhältnis Alkohol zu Carbonsäurestrom (Massenverhältnis) kann von 0,1 bis 30, bevorzugt 0,2 bis 20, besonders bevorzugt 0,5 bis 10 betragen.

Dieses Gemisch gelang als Schmelze oder Lösung in den Reaktor der Stufe 2, in dem die Carbonsäuren mit dem Alkohol verestert werden. Die Veresterungsreaktion kann bei 50 bis 400°C, bevorzugt 70 bis 300°C, besonders bevorzugt 90 bis 200°C durchgeführt werden. Es kann ein äußerer Druck angelegt werden, bevorzugt wird die Veresterung aber unter Eigendruck des Reaktionssystems durchgeführt. Als Veresterungsapparat kann dabei ein Rührkessel oder Strömungsrohr oder es können jeweils mehrere verwendet werden. Die für die Veresterung notwendige Verweilzeit liegt zwischen 0,3 und 10 Stunden, bevorzugt 0,5 bis 5 Stunden. Die Veresterungsreaktion kann ohne Zusatz eines Katalysators ablaufen, bevorzugt wird aber zur Erhöhung der Reaktionsgeschwindigkeit ein Katalysator zugesetzt. Dabei kann es sich um einen homogenen gelösten oder um einen festen Katalysator handeln. Als homogene Katalysatoren seien beispielhaft Schwefelsäure, Phosphorsäure, Salzsäure, Sulfonsäure wie p-Toluolsulfonsäure, Heteropolysäuren wie Wolframatophosphorsäure oder Lewissäuren wie z.B. Aluminium-, Vanadium-, Titan-, Bor-Verbindungen genannt. Bevorzugt sind Mineralsäuren, insbesondere Schwefelsäure. Das Gewichtsverhältnis von homogenem Katalysator zu Carbonsäureschmelze beträgt in der Regel 0,0001 bis 0,5, bevorzugt 0,001 bis 0,3.

Als feste Katalysatoren sind saure oder supersaure Materialien, z.B. saure oder supersaure Metalloxide wie SiO₂, Al₂O₃, SnO₂, ZrO₂, Schichtsilikate oder Zeolithe, die alle zur Säureverstärkung mit Mineralsäureestern wie Sulfat oder Phosphat dotiert sein können, oder organische Ionentauscher mit Sulfonsäure- oder Carbonsäuregruppen geeignet. Die festen Katalysatoren können als Festbett angeordnet oder als Suspension eingesetzt werden.

Das bei der Reaktion gebildete Wasser wird zweckmäßig kontinuierlich z.B. durch eine Membran oder destillativ entfernt.

Die Vollständigkeit des Umsatzes der in der Carbonsäureschmelze vorhandenen freien Carboxylgruppen wird mit der nach der Reaktion gemessenen Säurezahl (mg KOH/g) festgestellt. Sie beträgt abzüglich der gegebenenfalls zugesetzten Säure als Katalysator 0,01 bis 50, bevorzugt 0,1 bis 10. Dabei müssen nicht alle im System vorhandenen Carboxylgruppen als Ester des eingesetzten Alkohols vorliegen, sondern ein Teil kann in Form von dimeren oder oligomeren Estern mit dem OH-Ende der Hydroxycapronsäure vorliegen.

Das Veresterungsgemisch wird in Stufe 3, ein Membransystem oder bevorzugt eine Destillationskolonne, eingespeist. Wurde zur Veresterungsreaktion eine gelöste Säure als Katalysator eingesetzt, wird das Veresterungsgemisch zweckmäßig mit einer Base neutralisiert, wobei pro Säureäquivalent des Katalysators 1 bis 1,5 Basenäquivalente zugesetzt werden. Als Basen werden in der Regel Alkali- oder Erdalkalimetalloxide, - Carbonate-, -Hydroxyde oder-Alkoholate, oder Amine in Substanz oder in dem Veresterungsalkohol gelöst verwendet. Es kann jedoch auch mit basischen Ionentauschern neutralisiert werden.

Wird in Stufe 3 eine Kolonne verwendet, so erfolgt der Zulauf der Kolonne bevorzugt zwischen dem Kopf- und dem Sumpfstrom. Über Kopf wird bei Drücken von 1 bis 1500 hPa, bevorzugt 20 bis 1000 hPa, besonders bevorzugt 40 bis 800 hPa und Temperaturen zwischen 0 und 150°C, bevorzugt 15 und 90°C und insbesondere 25 und 75°C der überschüssige Veresterungsalkohol ROH, Wasser sowie entsprechende Ester der Ameisensäure, Essigsäure und Propionsäure abgezogen. Dieser Strom kann entweder verbrannt oder bevorzugt in der Stufe 11 weiter aufgearbeitet werden.

Als Sumpf wird ein Estergemisch erhalten, das vorwiegend aus den Estern des eingesetzten Alkohols ROH mit Dicarbonsäuren wie Adipinsäure und Glutarsäure, Hydroxycarbonsäuren wie 6-Hydroxycapronsäure und 5-Hydroxyvaleriansäure sowie aus Oligomeren und freien bzw. veresterten 1,4-Cyclohexandiolen besteht. Es kann sinnvoll sein, einen Restgehalt von Wasser und/oder Alkohol ROH bis je 4 Gew.-% im Estergemisch zuzulassen. Die Sumpftemperaturen betragen 70 bis 250°C, bevorzugt 80 bis 220°C, besonders bevorzugt 100 bis 190°C.

Der weitgehend von Wasser und Veresterungsalkohol ROH befreite Strom aus Stufe 3 wird in die Stufe 4 eingespeist. Dabei handelt es sich um eine Destillationskolonne, bei der der Zulauf zwischen den leichtsiedenden Komponenten und den schwersiedenden Komponenten erfolgt. Die Kolonne wird bei Temperaturen von 10 bis 300°C, bevorzugt 20 bis 270°C, besonders bevorzugt 30 bis 250°C und Drücken von 1 bis 1000 hPa abs., bevorzugt 5 bis 500 hPa, besonders bevorzugt 10 bis 200 hPa betrieben.

Die Kopffraktion besteht überwiegend aus Restwasser und Restalkohol ROH, Estern des Alkohols ROH mit Monocarbonsäuren, überwiegend C₃- bis C₆-Monocarbonsäureestern mit Hydroxycarbonsäuren, wie 6-Hydroxycapronsäure, 5-Hydroxyvaleriansäure sowie vor allem den Diestern mit Dicarbonsäuren wie Adipinsäure, Glutarsäure und Bernsteinsäure, Cyclohexandiolen, ε-Caprolacton und Valerolacton.

Die genannten Komponenten können zusammen über Kopf abgetrennt oder in einer weiteren bevorzugten Ausführungsform in der Kolonne der Stufe 4 in einem Kopfstrom, der überwiegend Restwasser und Restalkohol sowie die oben erwähnten Bestandteile mit 3 bis 5 C-Atomen enthält und einen Seitenstrom, der überwiegen die oben erwähnten Bestandteile der C₆-Ester enthält, aufgetrennt werden. Der die Ester der C₆-Säuren enthaltende Strom, entweder als Gesamt-Kopfstrom oder als Seitenstrom kann dann, je nachdem wie viel ε-Caprolacton hergestellt werden soll, gemäß dem nach WO 97/31883 bevorzugten Verfahren nur zum Teil oder als Gesamtstrom in die Stufe 12 eingespeist werden.

Die schwersiedenden Komponenten des Stromes aus Stufe 4, überwiegend bestehend aus dimeren oder oligomeren Estern, Cyclohexandiolen sowie nicht näher definierte z. T. polymeren Bestandteilen der DCL, werden über den Abtriebsteil der Kolonne der Stufe 4 abgetrennt, können entweder verbrannt werden oder in einer bevorzugten Ausführungsform zur so genannten Umesterung in die in WO 97/31883 beschriebenen Stufe 8 gelangen.

Die Stufen 3 und 4 können, insbesondere wenn nur kleinere Mengen verarbeitet werden, zusammengefasst werden. Dazu kann beispielsweise in einer absatzweise durchgeführten fraktionierten Destillation der C₆-Esterstrom gewonnen werden.

Für die ε-Caprolacton-Herstellung wird der vorwiegend Ester der C₆-Säuren enthaltende Strom aus der Stufe 4 eingesetzt. Dazu wird dieser Strom in Stufe 12, einer Destillationskolonne, in einen überwiegend Adipinsäurediester enthaltenden Strom über Kopf und einen überwiegend 6-Hydroxycapronsäureester enthaltenden Strom über Sumpf abgetrennt. Die Kolonne wird bei Drücken von 1 bis 500 hPa abs., bevorzugt 5 bis 350 hPa, besonders bevorzugt 10 bis 200 hPa und Sumpftemperaturen von 80 bis 250°C, bevorzugt 100 bis 200°C, besonders bevorzugt 110 bis 180°C betrieben. Die Kopftemperaturen stellen sich dabei entsprechend ein.

Wichtig für eine hohe Reinheit und hohe Ausbeute an ε-Caprolacton ist die Abtrennung der 1,2-Cyclohexandiole vom Hydroxycapronsäureester, da diese Komponenten Azeotrope miteinander bilden. Es war in dieser Stufe 12 nicht vorauszusehen, dass die Trennung der 1,2-Cyclohexandiole und des Hydroxycapronsäureesters vollständig gelingt, vor allem wenn als Ester der bevorzugte Methylester eingesetzt wird.

Es kann vorteilhaft sein, in der Stufe 12 zusammen mit dem Adipinsäurediester auch etwas Hydroxycapronsäureester abzutrennen. Die Gehalte des Adipinsäureesters an Hydroxycapronsäureester liegen, wenn der Adipinsäurediester zu 1,6-Hexandiol hydriert werden soll, dabei vorteilhaft zwischen 0,2 und 7 Gew.-%. Je nach Alkoholkomponente der Ester wird dieser Anteil Hydroxycapronsäureester zusammen mit dem Adipinsäurediester über Kopf (z.B. Methylester) oder über Sumpf (z.B. Butylester) abgetrennt.

Der 6-Hydroxycapronsäureester enthaltende Strom wird in der Flüssigphase zu Alkohol und ε-Caprolacton umgesetzt. Er kann noch weitere Komponenten enthalten, die einen Gewichtsanteil von bis zu 20 % ausmachen können, bevorzugt aber unter 10 % Anteil liegen, besonders bevorzugt unter 5 %. Diese Komponenten bestehen z.B. aus 1,5-Pentandiol, Cyclohexandiolen, ungesättigten Adipinsäurediestern, Pimelinsäurediestern, ε-Caprolacton, 5-Hydroxycapronsäureester sowie Diestern auf Basis u. a. von 6-Hydroxycapronsäure-estern.

Die Reaktion wird ohne Katalysator oder aber bevorzugt in Anwesenheit eines Katalysators durchgeführt. Als Katalysatoren eignen sich saure oder basische Katalysatoren, die homogen gelöst oder heterogen vorliegen können. Beispiele sind Alkali- und Erdalkalimetallhydroxide, -oxide, -carbonate, -alkoxylate, oder -carboxylate, Säuren wie Schwefel- oder Phosphorsäure, organische Säuren wie Sulfonsäuren oder Mono- oder Dicarbonsäuren, bzw. Salze der vorgenannten Säuren, Lewissäuren oder Lewisbasen, bevorzugt aus der III. und IV. Hauptgruppe bzw. der I. bis VIII. Nebengruppe des Periodensystems der Elemente.

Bevorzugt verwendet man die gleichen Katalysatoren, die auch in der Stufe 8 eingesetzt werden, da der hochsiedende Ausschleusstrom der Stufe 13 oligomere Hydroxycapronsäureeinheiten enthält, die vorteilhaft über die Stufe 8 wieder verwertet werden können. Wird ein heterogener Katalysator eingesetzt, so beträgt die Katalysatorbelastung üblicherweise 0,05 bis 5 kg Edukt/I Katalysator und Stunde. Bei homogenen Katalysatoren wird der Katalysator bevorzugt dem Eduktstrom zugemischt. Dabei beträgt die Konzentration üblicherweise 10 bis 10000 ppm, bevorzugt 50 bis 5000 ppm, besonders bevorzugt 100 bis 1000 ppm. Die Reaktion wird üblicherweise bei 150 bis 400°C, bevorzugt 180 bis 350°C, besonders bevorzugt 190 bis 330°C und Drücken von 1 bis 1020 hPa, bevorzugt 5 bis 500 hPa, besonders bevorzugt 10 bis 200 hPa durchgeführt.

In manchen Fällen ist es vorteilhaft, die Cyclisierungs-Reaktion in Gegenwart von hochsiedenden Mono-, Di- oder Polyolen wie z.B. Decanol, Undecanol, Tridecanol, Pentadecanol, Octadecanol, 1,4-Butandiol, 1,5-Pentandiol, 1,6-Hexandiol, 1,4-Cyclohexandiolen, Butylethylpropandiol, Neopentylglycol, Triethylenglykol, Tetraethylenglykol, Trimethylolpropan oder Glycerin durchzuführen.

Diese hochsiedenden Alkohole oder Polyole werden vorgelegt und/oder dem Reaktionsgemisch zugesetzt oder separat zudosiert z.B. jeweils in Konzentrationen von 0,1 bis 90 Gew.% bevorzugt 1 bis 60 Gew.%, besonders bevorzugt 5 bis 30 Gew.-%.

Die Reaktionsprodukte, vorwiegend Veresterungsalkohol ROH und ε-Caprolacton, werden vorwiegend gasförmig aus dem Reaktionsgemisch entfernt. Vorteilhaft ist eine dem Reaktionsgefäß aufgesetzte Kolonne, in der noch nicht umgesetztes Edukt im Reaktionssystem gehalten werden kann und über Kopf der Alkohol und ε-Caprolacton abgezogen werden. Dabei kann die Kondensation des Produktstroms so erfolgen, dass fraktioniert kondensiert wird, d. h. zuerst vorwiegend ε-Caprolacton, dann der Veresterungsalkohol. Selbstverständlich kann auch nur der Alkohol über Kopf, ε-Caprolacton dagegen in einem Seitenstrom gewonnen werden. Der Alkohol-Strom kann vorteilhaft in die Stufe 2, 8 oder 11 zurückgeführt werden. Das Sumpfprodukt der Cyclisierung besteht aus einem komplexen Gemisch aus Oligomeren und Polymeren.

Der Zulauf zum Reaktionsgefäß kann ohne Vorheizung erfolgen. Werden homogene Katalysatoren verwendet, so ist es vorteilhaft, den Eduktstrom direkt in den Cyclisierungssumpf einzutragen. Dabei kann der Katalysator entweder schon vor der Reaktion dem Feed zugefügt werden oder direkt in das Reaktionsgefäß gegeben werden.

Vorteilhafter ist es jedoch den Zulauf vorzuheizen, vor allem, wenn der Katalysator bereits gelöst ist und ein Hydroxycapronsäureester mit einer C₁-C₅-Alkoholkomponente verwendet wird. Die Vorheiztemperatur liegt dabei zwischen 100 und 300°C, bevorzugt bei 130 bis 270°C, besonders bevorzugt bei 150 bis 250°C. Bei diesen Temperaturen reagiert der Hydroxycapronsäureester bereits zum Teil zu Alkohol, ε-Caprolacton und dimeren bzw. oligomeren Hydroxycapronsäureestern. Dies bewirkt, dass nur wenig Hydroxycapronsäureester, wenn er in das heiße Reaktionsgefäß gelangt, sofort aus dem Reaktionssumpf abdestillieren kann. Auf diese Weise werden Kolonnenböden eingespart.

Eine weitere vorteilhafte Möglichkeit besteht darin, den überwiegenden Teil des Veresterungsalkohols vor der Aufarbeitung des ε-Caprolacton zu gewinnen, vor allem wenn dieser Alkohol, wie Methanol, niedrig siedet und in der Folge nur aufwendig kondensierbar wäre. Dazu wird der Hydroxycapronsäuremethylester in Gegenwart eines Katalysators wie oben beschrieben vorerhitzt, wobei bereits der freiwerdende Alkohol abdestilliert. Dies geschieht vorteilhaft bei 100 bis 1100 hPa abs., einem Druckbereich, bei dem der Esteralkohol leicht kondensierbar ist. Dieses Vorgehen ist bevorzugt in Gegenwart der oben beschriebenen hochsiedenden Alkohole möglich.

Erfindungsgemäß wird das nicht flüchtige Sumpfprodukt der 6-Hydroxycapronsäureester-Cyclisierung in einem Reaktionsgefäß bei vermindertem Druck diskontinuierlich oder kontinuierlich einer thermischen Behandlung unterworfen.

Der Kopfstrom der Cyclisierung (Fig. 1, Stufe 13) wird nach Kondensation in Stufe 14 weiter aufgearbeitet. Dabei kann es sich um eine oder mehrere Kolonnen handeln. Wird eine Kolonne verwendet, so werden über Kopf der gegebenenfalls noch vorhandene Veresterungsalkohol sowie andere C₁- bis C₆-Leichtsieder abgetrennt, über Seitenstrom reines ε-Caprolacton und über den Sumpf gegebenenfalls noch nicht umgesetzter Hydroxycapronsäureester, der zurückgeführt wird.

Hochreines ε-Caprolacton erhält man, wenn in der Stufe 14 die erwähnten Leichtsieder in einer ersten Kolonne über Kopf, ε-Caprolacton und andere Hochsieder über Sumpf in eine zweite Kolonne eingespeist werden, wo ε-Caprolacton über Kopf abgezogen wird. Handelt es sich bei dem zu gewinnenden ε-Caprolactonstrom nur um kleinere Mengen, so kann ε-Caprolacton mit einer Kolonne durch absatzweise fraktionierte Destillation gewonnen werden.

Die kondensierten Kopfprodukte der thermischen Behandlung, die unter Bedingungen der thermischen Behandlung flüchtigen Verbindungen, die hauptsächlich ε-Caprolacton enthalten, können destillativ in gleicher Weise wie die kondensierten Kopfprodukte der Cyclisierung, die unter Bedingungen der Cyclisierung flüchtigen Verbindungen, zu hochreinem ε-Caprolacton aufgearbeitet werden.

Dabei kann es sinnvoll sein, die beiden Kopfprodukte zu vereinigen und gemeinsam, wie beschrieben, destillativ aufzuarbeiten.

Es kann aber auch vorteilhaft sein, die beiden Kopfprodukte getrennt zu hochreinem ε-Caprolacton aufzuarbeiten.

Die ein- oder mehrstufigen Destillationen zur Reinigung des Caprolactons werden bei Sumpftemperaturen von 70 bis 250°C, bevorzugt 90 bis 230°C, besonders bevorzugt 100 bis 210°C und Drücken von 1 bis 500 hPa abs., bevorzugt 5 bis 200 hPa, besonders bevorzugt 10 bis 150 hPa durchgeführt.

Das Verfahren wird anhand der nachfolgenden Beispiele näher erläutert, aber dadurch in keiner Weise beschränkt.

### Beispiele

### Beispiel 1

### Cyclisierung

1000 g/h eines Gemisches aus ca. 93 %- 6-Hydroxycapronsäuremethylester, 1,0 % Adipinsäuredimethylester, 1,6% 1,4-Cyclohexandiole, 1,4% 1,5-Pentandiol, 0,3% ungesättigte Adipinsäuredimethylester, 0,2 % Pimelinsäuredimethylester, 1,6% dimeren Ester sowie weitere Verbindungen, die mengenmäßig jeweils unter 0,1 % vorlagen, hergestellt entsprechend WO 97/31 883, wurden zusammen mit 1000 ppm Titanat (Gemisch aus iso-Propyl (80%)- und n-Butyltitanaten (20 %)) sowie 50 g/h 1,6-Hexandiol in einen 5 Liter-Reaktor mit aufgesetzter Kolonne zur Cyclisierung gepumpt. Der Stand im Reaktor wurde mittels Regelung bei ca. 40 % gehalten. Überschüssiger Reaktorinhalt wurde ausgeschleust. Der Reaktorinhalt wurde umgepumpt, wobei im Umpumpkeis ein Wärmetauscher dem System Energie zuführte. Bei einer Reaktortemperatur von ca. 220°C und einem Druck von 40 hPa absolut destillierte bei einem Rücklaufverhältnis von 5 : 1 pro Stunde überwiegend Methanol und ε-Caprolacton ab. Der Destillatstrom wurde bei ca. 10°C kondensiert. Die gaschromatographischer Analyse ergab eine Ausbeute an ε-Caprolacton von 65 Mol.%.

### Thermische Behandlung

Der flüssige Reaktionsaustrag aus dem Cyclisierungsreaktor (das Sumpfprodukt der Cyclisierung) wurde gesammelt und absatzweise unter Zusatz von weiteren 1000 ppm Titanat in einer Destillationsblase mit 2 Litern Inhalt sowie aufgesetzter Kolonne (Rücklaufverhältnis 1 : 1) bei 250°C und 10 hPa absolut für 3 Stunden erhitzt. Das dabei anfallende Destillationsprodukt enthielt dabei überwiegend ε-Caprolacton sowie 1,6-Hexandiol. Die molare ε-Caprolactonausbeute betrug in dieser zweiten Reaktionsstufe in den einzelnen Umsetzungen um 25 % ε-Caprolacton, so dass über das gesamte Verfahren eine Ausbeute von 90 Mol% erreicht wurde. Das in der Cyclisierung und der thermischen Behandlung anfallende Produkt wurde absatzweise bei ca. 40 mbar destilliert. Dabei wurde Caprolacton mit einer Reinheit von 99,90 % erhalten.

### Beispiel 2

### Thermische Behandlung

Beispiel 1 wurde wiederholt, mit dem Unterschied, dass in der absatzweise durchgeführten thermische Behandlung pro kg flüssiger Reaktionsaustrag aus dem Cyclisierungreaktor 0,2 kg C₁₅-Alkohol zugegeben wurde. Dadurch konnte die Caprolactonausbeute in der thermischen Behandlung auf ca. 30 % gesteigert werden. Die Reindestillation analog Beispiel 1 ergab eine Caprolactonreinheit von 99,92 %

### Beispiel 3

### Cyclisierung

20 kg des 6-Hydroxycapronsäure-haltigen Edukts gemäß Beispiel 1 wurden mit 5 kg 1,5-Pentandiol vermischt und nach Zugabe von 1000 ppm Titanat (Gemisch aus iso-Propyl- und n-Butyltitanaten) bei Normaldruck auf 180°C für 5 Stunden erhitzt. Während dieser Zeit destillierte überwiegend Methanol ab. Der verbleibende Rückstand wurde kontinuierlich bei 20 mbar absolut und 240°C in einen Wischblattverdampfer (Sambay) gefördert. Das dabei entstehende Destillat enthielt überwiegend Caprolacton und 1,5-Pentandiol. Die Ausbeute an Caprolaton lag bei ca. 80 Mol%.

### Thermische Behandlung

Das verbliebene Sumpfprodukt der Cyclisierung wurde in einen 5 Liter-Reaktor mit aufgesetzter Kolonne thermisch behandelt, nachdem nochmals 1000 ppm Titanat zugegeben worden waren. Der Reaktorinhalt wurde umgepumpt, wobei im Umpumpkeis ein Wärmetauscher dem System Energie zuführte. Bei einer Reaktortemperatur von ca. 230°C und einem Druck von 10 hPa absolut destillierte bei einem Rücklaufverhältnis von 1 : 1 pro Stunde überwiegend ε-Caprolacton ab. In dem daraus resultierenden Destillat lagen noch weitere 12 Mol% Caprolacton vor. Die Reindestillation analog Beispiel 1 ergab eine Caprolactonreinheit von 99,89 %.

## Patentansprüche

1. Verfahren zur Herstellung von ε-Caprolacton in einer Reinheit über 99 % durch Cyclisierung von 6-Hydroxycapronsäureester in der Flüssigphase bei 150 bis 400°C und 1 bis 1020 hPa abs., Abtrennung und Kondensation der unter Cyclisierungsbedingungen flüchtigen Verbindungen, **dadurch gekennzeichnet, dass** das zurückbleibende Sumpfprodukt der Cyclisierung in mindestens einem weiteren Reaktor thermisch in Gegenwart von 0,1 bis 90 Gew.-% Mono-, Di- oder Polyolen behandelt wird, flüchtige Verbindungen abgetrennt und kondensiert werden und ε-Caprolacton durch Destillation aus den Kondensaten gewonnen wird.

2. Verfahren zur Herstellung von ε-Caprolacton in einer Reinheit über 99 % nach Anspruch 1, **dadurch gekennzeichnet, dass** die thermische Behandlung des Sumpfproduktes der Cyclisierung bei bis 100°C über der Cyclisierungstemperatur liegenden Temperaturen durchgeführt wird.

3. Verfahren zur Herstellung von ε-Caprolacton in einer Reinheit über 99 % nach Anspruch 1 oder 2, **dadurch gekennzeichnet, dass** bei einem Druck unter 50 hPa thermisch behandelt wird.

4. Verfahren zur Herstellung von ε-Caprolacton in einer Reinheit über 99 % nach einem der Ansprüche 1 bis 3, **dadurch gekennzeichnet, dass** das Sumpfprodukt der Cyclisierung 0,1 bis 24 h thermisch behandelt wird.

5. Verfahren zur Herstellung von ε-Caprolacton in einer Reinheit über 99 % nach Ansprüche 1 bis 4, **dadurch gekennzeichnet, dass** die thermische Behandlung kontinuierlich oder diskontinuierlich durchgeführt wird.

6. Verfahren zur Herstellung von ε-Caprolacton in einer Reinheit über 99 % nach nach einem der Ansprüche 1 bis 5, bei dem ein Adipinsäure, 6-Hydroxycapronsäure und geringe Mengen 1,4-Cyclohexandiole enthaltendes Carbonsäuregemisch, das als Nebenprodukt der Oxidation von Cyclohexan zu Cyclohexanon/Cyclohexanol mit Sauerstoff oder Sauerstoff enthaltenden Gasen durch Wasserextraktion des Reaktionsgemisches erhältlich ist, mit einem niedermolekularen Alkohol zu den entsprechenden Carbonsäureestern verestert wird, das so erhaltene Veresterungsgemisch in mindestens einer Destillationsstufe so aufgetrennt wird, dass man den 6-Hydroxycapronsäureester enthaltenden Strom erhält.

7. Verfahren zur Herstellung von ε-Caprolacton in einer Reinheit über 99 % nach Anspruch 6, bei dem zur Herstellung des 6-Hydroxycapronsäuremethylester
- das erhaltene Veresterungsgemisch in einer ersten Destillationsstufe von überschüssigem Methanol und Leichtsiedern befreit wird,
- aus dem Sumpfprodukt in einer zweiten Destillationsstufe eine Auftrennung in eine von 1,4-Cyclohexandiolen im Wesentlichen freie Esterfraktion und eine zumindest den größeren Teil der 1,4-Cyclohexandiole enthaltende Fraktion durchgeführt wird,
- der 6-Hydroxycapronsäuremethylester-Strom von der Esterfraktion in einer dritten Destillationsstufe abgetrennt wird.

8. Verfahren zur Herstellung von ε-Caprolacton in einer Reinheit über 99 % nach einem der Ansprüche 1 bis 7 **dadurch gekennzeichnet, dass** die Cyclisierung in Gegenwart von 1,6-Hexandiol durchgeführt wird.

## Claims

1. A process for preparing ε-caprolactone in a purity of more than 99% by cyclizing 6-hydroxycaproic esters in the liquid phase at from 150 to 400°C and from 1 to 1020 hPa abs., and removing and condensing the compounds which are volatile under cyclization conditions, which comprises thermally treating the remaining bottom product of the cyclization in at least one further reactor in the presence of from 0.1 to 90% by weight of mono-, di- or polyols, removing and condensing volatile compounds and obtaining ε-caprolactone by distillation from the condensates.

2. The process for preparing ε-caprolactone in a purity of more than 99% according to claim 1, wherein the thermal treatment of the bottom product of the cyclization is performed at temperatures up to 100°C above the cyclization temperature.

3. The process for preparing ε-caprolactone in a purity of more than 99% according to claim 1 or 2, wherein thermal treatment is effected at a pressure below 50 hPa.

4. The process for preparing ε-caprolactone in a purity of more than 99% according to any of claims 1 to 3, wherein the bottom product of the cyclization is thermally treated for from 0.1 to 24 h.

5. The process for preparing ε-caprolactone in a purity of more than 99% according to claims 1 to 4, wherein the thermal treatment is performed continuously or batchwise.

6. The process for preparing ε-caprolactone in a purity of more than 99% according to any of claims 1 to 5, in which a carboxylic acid mixture which comprises adipic acid, 6-hydroxycaproic acid and small amounts of 1,4-cyclohexanediols and is obtainable as a by-product of the oxidation of cyclohexane to cyclohexanone/cyclohexanol with oxygen or oxygen-comprising gases by water extraction of the reaction mixture is esterified with a low molecular weight alcohol to give the corresponding carboxylic esters, the esterification mixture thus obtained is separated in at least one distillation stage so as to obtain the stream comprising 6-hydroxycaproic esters.

7. The process for preparing ε-caprolactone in a purity of more than 99% according to claim 6, in which methyl 6-hydroxycaproate is prepared by
- freeing the resulting esterification mixture of excess methanol and low boilers in a first distillation stage,
- from the bottom product, in a second distillation stage, performing a separation into an ester fraction essentially free of 1,4-cyclohexanediols and a fraction comprising at least the majority of the 1,4-cyclohexanediols,
- removing the methyl 6-hydroxycaproate stream from the ester fraction in a third distillation stage.

8. The process for preparing ε-caprolactone in a purity of more than 99% according to any of claims 1 to 7, wherein the cyclization is performed in the presence of 1,6-hexanediol.

## Revendications

1. Procédé pour la préparation d'ε-caprolactone à une pureté supérieure à 99% par cyclisation d'esters de l'acide 6-hydroxycaproïque en phase liquide à 150 jusqu'à 400°C et à une pression de 1 à 1020 hPa abs., séparation et condensation des composés volatils dans les conditions de cyclisation, **caractérisé en ce que** le produit du fond résiduel de la cyclisation est traité dans au moins un autre réacteur, thermiquement, en présence de 0,1 à 90% en poids de mono-ols, de diols ou de polyols, les composés volatils sont séparés et condensés et l'ε-caprolactone est obtenue par distillation à partir des condensats.

2. Procédé pour la préparation d'ε-caprolactone à une pureté supérieure à 99% selon la revendication 1, **caractérisé en ce que** le traitement thermique du produit du fond de la cyclisation est réalisé à des températures situées à jusqu'à 100°C au-dessus de la température de cyclisation.

3. Procédé pour la préparation d'ε-caprolactone à une pureté supérieure à 99% selon la revendication 1 ou 2, **caractérisé en ce qu'**on traite thermiquement à une pression inférieure à 50 hPa.

4. Procédé pour la préparation d'ε-caprolactone à une pureté supérieure à 99% selon l'une quelconque des revendications 1 à 3, **caractérisé en ce que** le produit du fond de la cyclisation est traité thermiquement pendant 0,1 à 24 h.

5. Procédé pour la préparation d'ε-caprolactone à une pureté supérieure à 99% selon l'une quelconque des revendications 1 à 4, **caractérisé en ce que** le traitement thermique est réalisé de manière continue ou discontinue.

6. Procédé pour la préparation d'ε-caprolactone à une pureté supérieure à 99% selon l'une quelconque des revendications 1 à 5, dans lequel on estérifie un mélange d'acides carboxyliques contenant de l'acide adipique, de l'acide 6-hydroxycaproïque et de faibles quantités de 1,4-cyclohexanediols, qui peut être obtenu comme produit secondaire de l'oxydation de cyclohexane en cyclohexanone/cyclohexanol avec de l'oxygène ou des gaz contenant de l'oxygène par extraction à l'eau du mélange réactionnel, avec un alcool de bas poids moléculaire, en esters correspondants des acides carboxyliques, le mélange d'estérification ainsi obtenu est séparé, dans au moins une étape de distillation, de manière telle qu'on obtient le flux contenant l'ester de l'acide 6-hydroxycaproïque.

7. Procédé pour la préparation d'ε-caprolactone à une pureté supérieure à 99% selon la revendication 6, dans lequel, pour la préparation de l'ester méthylique de l'acide 6-hydroxycaproïque,
- on libère le mélange d'estérification obtenu, dans une première étape de distillation, du méthanol en excès et des fractions légères,
- on réalise sur le produit du fond, dans une deuxième étape de distillation, une séparation en une fraction d'ester pratiquement exempte de 1,4-cyclohexanediols et en une fraction contenant au moins la plus grande partie des 1,4-cyclohexanediols,
- on sépare, dans une troisième étape de distillation, le flux contenant l'ester méthylique de l'acide 6-hydroxycaproïque de la fraction d'ester.

8. Procédé pour la préparation d'ε-caprolactone à une pureté supérieure à 99% selon l'une quelconque des revendications 1 à 7, **caractérisé en ce que** la cyclisation est réalisée en présence de 1,6-hexanediol.
